(19) Europäisches Patentamt
European Patent Office
Office européen des brevets

(11) **EP 3 751 578 A1**

(12) **EUROPEAN PATENT APPLICATION**

(43) Date of publication:
**16.12.2020 Bulletin 2020/51**

(51) Int Cl.:
***G16H 20/40*** *(2018.01)*

(21) Application number: **19179596.2**

(22) Date of filing: **12.06.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **DA SILVA, Ikaro Garcia Araujo**
**5656 AE Eindhoven (NL)**
• **BUIZZA, Roberto**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **CARDIOPULMONARY RESUSCITATION ASSESSMENT SYSTEM, METHOD AND COMPUTER PROGRAM**

(57)     According to an aspect, there is provided a cardiopulmonary resuscitation, CPR, system (1) for assessing chest compressions applied to a patient (5) by a responder (6) during delivery of CPR to the patient (5). The system (1) comprises: a sensor (2) configured to acquire sensor data indicating movement of the chest of the patient (5) due to a chest compression applied by the responder (6) during CPR delivery; a processor (3) configured to determine a force profile of the chest compression applied by the responder (6) during CPR delivery using the acquired sensor data; and an output (4) configured to output a message so as to communicate the message to the responder (6). The processor (3) is configured to determine the message to be communicated to the responder (6) in accordance with the force profile. According to other aspects, there is provided a method of assessing chest compressions applied to a patient (5) by a responder (6) during delivery of cardiopulmonary resuscitation, CPR, to the patient (5), and a computer program which, when executed on a computing system, carries out a method of assessing chest compressions applied to a patient (5) by a responder (6) during delivery of cardiopulmonary resuscitation, CPR, to the patient (5).

Fig. 4

**Description**

FIELD OF THE INVENTION

Embodiments of the present invention relate generally to cardiopulmonary resuscitation (CPR) and to a system, a method and a corresponding computer program for assessing chest compressions applied to a patient by a responder during delivery of CPR to the patient.

BACKGROUND OF THE INVENTION

The general background of this invention is in cardiopulmonary resuscitation (CPR) and a CPR system to assess chest compressions applied to the chest of a patient and assist with the delivery of CPR to the patient. CPR involves a responder (rescuer) applying chest compressions to a patient so as to manually pump oxygenated blood to the brain. The effectiveness of chest compressions delivered during CPR can vary depending on a number of factors, such as, for example the magnitude and direction of the force applied by the responder. CPR systems may be used to aid the responder with the delivery of CPR to the patient and thus increase the effectiveness of the CPR delivered to the patient. It is desirable to assess the application of chest compressions during CPR delivery to the patient by the responder so that the CPR may be more effectively applied and the benefit of CPR to the patient may be increased.

SUMMARY OF THE INVENTION

According to an embodiment of an aspect, there is provided a cardiopulmonary resuscitation, CPR, system for assessing chest compressions applied to a patient by a responder during delivery of CPR to the patient, the system comprising: a sensor configured to acquire sensor data indicating the movement of the chest of the patient due to a chest compression applied by the responder during CPR delivery; a processor configured to determine a force profile of the chest compression applied by the responder during CPR delivery using the acquired sensor data; and an output configured to output a message so as to communicate the message to the responder, wherein the processor is configured to determine the message to be communicated to the responder in accordance with the force profile.

Thus, according to embodiments of the present invention, a chest compression applied to the chest of the patient during CPR delivery may be assessed using sensor data indicative of the movement of the chest of the patient during the chest compression. The sensor data is used to determine a force profile of the chest compression and a message is determined based on the force profile. The message is output so that it is communicated to the responder. The direction and/or magnitude of the force applied by the responder may be represented by the force profile. The direction and/or magnitude of the applied force will likely vary due to the positioning/orientation of the arms of the responder delivering the chest compression and thus, by deriving a force profile from the acquired sensor data, the positioning/orientation of the arms of the responder during the chest compression with respect to the patient, particularly the chest of the patient, may be estimated from the sensor data. The sensor data indicative of the movement of the chest of the patient may therefore be used to assess the chest compression and the CPR delivery technique of the responder.

The force profile is representative of the force applied to the chest of the patient by the responder during the chest compression. The CPR system may therefore assess the force applied to the chest of a patient by a responder during the delivery of CPR and thus may assess the delivery of CPR to the patient. Using known information about CPR, such as the most effective direction to apply the force to the chest of the patient during the chest compression, the effectiveness of the assessed chest compression may also be derived. That is, the force profile derived from the measured sensor data may be used to estimate the effectiveness of the CPR delivered to the patient because the proper application of force for optimum CPR delivery is known.

A message that is determined based on a force profile of the chest compression derived from sensor data of the movement of the chest of the patient may therefore be communicated to the responder that delivered the chest compression. Accordingly, the chest compression may be assessed and feedback about the chest compression may be communicated to the responder. The message may, for example, indicate whether the force of the chest compression is appropriately applied to maximize the effectiveness of the chest compression.

By analyzing the chest compression and providing a corresponding message to the responder, subsequent chest compressions applied by the responder may be altered such that they result in more effective delivery of CPR. For example, if the determined force profile indicates that the force of the chest compression is not applied by the responder in the correct direction for optimum CPR delivery, then the processor may determine a message to be output to the responder that reflects this and instructs the responder to correct their chest compression technique. Then, after receipt of the message, the responder may adjust the direction of the force applied in subsequent chest compressions to improve the delivery of CPR.

When determining the message to be output, the processor may therefore determine, from the force profile, whether the force is applied in the desired direction and may then determine the message accordingly. That is, since the force profile indicates a relationship between the measured movement of the chest of the patient and the force applied by the responder, the processor may determine, from the force profile, whether the force is appropriately applied. The application of force in an incorrect direction may result from improper orientation or positioning of the arms of the responder. The determined message may therefore instruct the responder to adjust their arm orientation and/or positioning. For example, the message may instruct the responder to position their shoulders such that they are positioned above their hands when applying the chest compression, i.e. so that the hands and shoulders are vertically aligned.

The sensor data is representative of the movement of the chest of the patient during the chest compression. It may also be considered that the sensor data is indicative of or relates to the movement of the chest of the patient. Thus, from the movement of the chest, the force of the chest compression may be assessed and a force profile of the force of the applied chest compression may be determined to represent the assessed force. The sensor may comprise one or more sensors that are capable of measuring data indicative of the movement of the chest of the patient, such as, for example, an accelerometer, a force sensor, a gyroscope, etc.

During CPR and the application of force to the patient's chest by the responder to provide a chest compression, a compression cycle starts with no force being applied to the chest, continues with increasing application of force until a maximum compression depth is reached, and then as the force is released, returns to the starting point. A chest compression comprises one compression cycle and the compression cycle may therefore be determined from the sensor data.

The sensor may continuously acquire sensor data over a given period, at a certain point in time, or at a plurality of time points over a given period. For example, the given period may correspond to one or more compression cycles of the CPR delivery. The sensor may acquire the sensor data and provide it to the processor. All or only some of the sensor data may be provided to the processor and the sensor may continuously or periodically provide the sensor data to the processor.

The processor may be communicably coupled with the sensor and may receive the sensor data from the sensor. The processor may analyze the sensor data to determine the force profile of the chest compression. The processor may, for example, determine the force profile by analyzing the movement of the chest of the patient in one or more directions. The processor may also be referred to as a controller.

The processor may be configured to periodically re-determine the force profile using the most recently acquired sensor data. The processor may be configured to periodically re-determine the message to be communicated to the responder based on the re-determined force profile. The processor may therefore dynamically determine the message to be communicated to the responder based on the latest force profile derived from the latest the sensor data and thus the most recent chest compression. The message communicated to the responder may therefore be up-to-date and relevant to their current CPR technique and arm positioning.

The term patient may be used to describe an individual that is suffering, or is suspected of suffering, cardiac arrest, i.e. a sudden loss of blood flow resulting from the failure of the heart to effectively pump. The patient is therefore an individual to whom cardiopulmonary resuscitation (CPR), comprising chest compressions, is being administered. The patient may also be referred to as a victim or casualty.

The term responder may be used to describe an individual or rescuer that is delivering CPR (or at least the chest compressions of CPR) to the patient. The responder may be considered as an individual that uses the CPR system and the responder may position elements of the CPR system, such as, for example, the sensor, on the chest of the patient prior to starting CPR.

By analyzing the acquired sensor data indicative of the movement of the chest of the patient, the processor may determine the force profile of the chest compression which may indicate the direction and/or magnitude of the force applied during the chest compression. In particular, the force profile may indicate a direction of a force of the chest compression relative to gravity. That is, the force profile may indicate whether the direction of the force is aligned with gravity or to what extent the direction of the force is not aligned with gravity.

The desired direction of the force with respect to gravity may vary depending on the inclination of the patient. If the patient is lying flat on their back, then the desired direction of the force may be straight downward so that the direction of the force is aligned with gravity. If the patient is inclined, i.e. not lying on flat ground, then the desired direction of the force may not be aligned with gravity but may be in a direction that is straight downward toward the surface that the patient is lying on, with respect to the inclination of the patient.

It may be considered that a message communicated to the responder is delivered to the responder. The output may be any suitable output device capable of communicating a message intended for the responder. The message may, at least partially, comprise one or more of: an audio message; a video message; and a haptic message. The output may comprise one or more of: a speaker configured to communicate at least part of the message audibly; a video display configured to communicate at least part of the message visually; and a haptic output configured to communicate at least part of the message haptically. That is, the message may be output as audio data; video data; and/or a signal to control a haptic device such as a vibration motor, or any combination thereof. Thus, the message may be delivered to the responder in the form of audio, video, text, haptic, or another communication method. The message may be related to the delivery of chest compressions to the patient and may be related to the direction of the force applied by the responder and the positioning of the arms of the responder with respect to the chest of the patient.

The speaker may be considered to be an audio output device. The message may comprise an audio aspect which communicates an audio message to the respondent in accordance with the force profile. For example, the message may comprise an audible alarm to warn the responder that the chest compression is not optimally applied and/or may comprise an audible instruction which may instruct the responder to adjust the positioning of their arms.

The video display may comprise a display in the vicinity of a cardiac arrest event and text, video and/or images may be displayed on the display. For example, the video display may be a monitor in a hospital room or an ambulance. The video display may be an augmented/virtual reality display to deliver video data to the responder. The message may comprise a visual aspect which communicates a visual message to the respondent in accordance with the force profile. For example, the message may comprise a visual alarm, such as, for example, a pop-up window or notification on a user interface of the display, to warn the responder that the chest compression is not optimally applied and/or may comprise a visual instruction which may instruct the responder to adjust the positioning of their arms.

The haptic output may be a vibration element provided as part of a device for the assistance of CPR, such as a CPR puck to be positioned between the hands of the responder delivering CPR and the patient's chest. Such a puck may, for example, comprise a sensor of the CPR system according to embodiments of the present invention. Thus, the message may at least partially be output as a control signal for controlling the haptic output such that the message may be communicated to the responder haptically. For example, the message may comprise vibration pulses at a CPR puck which act as a haptic alarm to warn the responder that the chest compression is not optimally applied and/or may comprise a vibration message or sequence which may instruct the responder to adjust the positioning of their arms.

The output may be configured to: establish a connection with a user device associated with the responder; and transmit at least part of the message to the user device. That is, the output may transmit (part of) the message to a user device associated with the responder so that the user device may communicate the message to the responder. For example, the message may be output at the user device as audio data, display data and/or haptic data. The user device associated with the responder may be the responder's smartphone, smartwatch, wearable electronic device, etc.

The sensor may be a triaxial accelerometer configured to measure linear acceleration of the chest of the patient in three orthogonal directions. One of the three orthogonal directions of the triaxial accelerometer may correspond to a transverse plane of the patient. The transverse plane of the patient may also be referred to as an axial or horizontal plane and may be perpendicular to the coronal or frontal plane of the patient. The direction corresponding to the transverse plane of the patient may be considered as being aligned with gravity when the patient is lying on their back, on a flat surface. The three orthogonal axes comprise an x-axis, a y-axis and a z-axis; and the z-axis may be most closely aligned with gravity and the z-axis may be aligned with gravity when the patient is lying flat on their back. The triaxial accelerometer may therefore be positioned on the chest of the patient such that the z-axis aligns with transverse plane of the patient.

The processor may be configured to analyze the sensor data of the linear acceleration of the chest of the patient in the three orthogonal directions. The processor may determine the direction of the force of the chest compression with respect to the three orthogonal directions. The force profile may indicate a direction of the force of the chest compression with respect to the three orthogonal directions. That is, the force profile may indicate the direction of the force of the chest compression as a vector of the three-dimensional axes.

The processor may be configured to determine whether a magnitude of linear acceleration in a direction other than the direction corresponding to the transverse plane of the patient is below a first predetermined magnitude threshold. The processor may be configured to determine whether a magnitude of linear acceleration in a direction other than the direction corresponding to the transverse plane of the patient is above a second predetermined magnitude threshold, which is greater than the first predetermined magnitude threshold.

If the chest compression is properly applied, then the force will be in a direction aligned with the direction corresponding to the transverse plane of the patient. This direction will be aligned with gravity when the patient is lying flat on their back and corresponds to the z-axis. Thus, when the force is properly applied, the acceleration of the sensor will have greatest magnitude in this direction and minimal acceleration in the other directions. If the magnitude of acceleration in either of the other directions exceeds a certain level, then it may be concluded that the chest compression is not properly applied because the direction of the force is not predominantly downward. Since the magnitude of acceleration in each direction may be positive or negative, two magnitude thresholds may be required. The first predetermined magnitude threshold may correspond to a negative number and the second predetermined magnitude threshold may correspond to a positive number. It may therefore be considered that the magnitude of acceleration in the directions other than the corresponding to the transverse plane of the patient should remain close to zero and the first predetermined magnitude threshold and the second predetermined magnitude threshold may be set at suitable values either side of zero to detect deviation from the desired level.

Thus, by monitoring the magnitude of (linear) acceleration in the directions other than the direction corresponding to the transverse plane of the patient, an improper application of the chest compression may be detected.

The processor may be configured to determine a message indicating that the force of the chest compression is not optimally applied in response to the magnitude of linear acceleration in the direction other than the direction corresponding to the transverse plane of the patient being below the first predetermined magnitude threshold, or the magnitude of linear acceleration in the direction other than the direction corresponding to the transverse plane of the patient being above the second predetermined magnitude threshold. That is, if the processor determines that a magnitude of linear acceleration in a direction other than the direction corresponding to the transverse plane of the patient is below the first predetermined magnitude threshold or above the second predetermined magnitude threshold, the processor may determine a message to be communicated to the responder which indicates that the chest compression is not optimally applied. For example, the message may indicate that the force of the chest compression is applied in the wrong direction and the message may include a reminder of the direction of force that may result in optimum chest compression application, i.e. directly downward in a direction perpendicular to the surface that the patient is lying on.

The sensor may be configured to: measure the linear acceleration of the chest of the patient at a first time point; and may be configured to measure the linear acceleration of the chest of the patient at a second time point occurring later than the first time point. The processor may be configured to: determine a first relationship between the three orthogonal directions at the first time point; and may be configured to determine a second relationship between the three orthogonal directions at the second time point. The processor may be configured to: calculate a difference between the first relationship and the second relationship; and may be configured to determine whether the difference exceeds a predetermined difference threshold. The processor may be configured to: determine a message indicating that the force of the chest compression is not optimally applied in response to the difference exceeding the predetermined difference threshold.

That is, the processor may determine whether the relationship between the acceleration in the three directions deviates from an allowable range between two points in time. The relationship may be a ratio of the accelerations in the three orthogonal directions. The first time point may correspond to the start of the compression cycle, immediately before the responder applies force to the chest of the patient. The second time point may correspond to the mid-point in the compression cycle in which the maximum compression depth is reached and the maximum force is applied by the responder, immediately before the responder releases the applied force. If the force is properly applied, then the ratio between the acceleration in the three directions will be consistent throughout the chest compression. Thus, by determining whether the ratio immediately before the force is released is within an allowable range from the ratio immediately before the application of force, proper application of force and thus arm position of the responder may be determined.

In other words, a normal relationship between the three orthogonal directions may be determined at the first time point and it may be determined that the force is applied incorrectly if the relationship between the three orthogonal directions deviates from this normal relationship. A gravity vector or gravity bias may be determined from the normal relationship between the three directions. This may be particularly useful when the patient is not lying flat and the chest of the patient is inclined such that the correct direction of the applied force is not aligned with gravity. Determining the relationship between the acceleration in the three directions may allow for the determination of whether the force is applied in the direction corresponding to the transverse plane of the patient. The processor may estimate the deviation of the gravity vector from the z-axis, and account for this bias when determining the force profile. If the patient is not lying flat, then there will be a bias on the three axis corresponding to the magnitude of gravity and the processor may estimate the inclination of the patient from the gravity vector and account for that bias. The bias may be indicated by the relationship between the three orthogonal directions of acceleration.

The sensor may also comprise a triaxial gyroscope configured to measure angular acceleration of the chest of the patient about the transverse plane of the patient. The processor may be configured to: analyze the sensor data of the angular acceleration of the chest of the patient; and determine the direction of the force of the chest compression with respect to the angular acceleration and linear acceleration of the chest of the patient.

Thus, angular acceleration of the chest of the patient may also be used to determine the force profile and the direction of the force applied during the chest compression. The force profile may indicate linear and angular acceleration of the chest of the patient. The angular acceleration may be measured about or with respect to the transverse plane, such that the normal of the gyroscope (i.e. no angular acceleration) is aligned with the transverse plane of the patient. Angular acceleration measured by the gyroscope may indicate a non-linear application of force by the responder and/or damage to the chest of the patient. This may include, for example, a rotation, twist and/or scoop component in the motion of the chest compression by the responder, or an indication that the chest is collapsing in a circular pattern (such as, for example, bulging or buckling with the gyroscope positioned on the bulging/buckling side). Such angular acceleration measured by the sensor may be indicative of non-efficient force application and thus sub-optimum CPR delivery.

The angular acceleration data acquired by the gyroscope may also be used or integrated with the linear acceleration data acquired by the accelerometer to determine a more accurate total acceleration profile, which may be used to determine a more accurate force profile. Thus the accuracy of the force profile and the determined direction of the force of the chest compression may be improved by the use of angular acceleration data acquired by the gyroscope.

The sensor may comprise: an accelerometer configured to measure linear acceleration of the chest of the patient in a direction corresponding to a transverse plane of the patient. The sensor may comprise a pressure sensor configured to measure a magnitude of the force of the chest compression. The force profile may indicate linear acceleration of the chest of the patient in the direction corresponding to the transverse plane of the patient in accordance with the magnitude of the force.

In other words, the sensor may comprise an accelerometer and a pressure sensor and the sensor data acquired from these sensors may be used to determine the force profile. The processor may use the linear acceleration in accordance with the magnitude of the force to determine the force profile.

The pressure sensor may measure, as pressure sensor data, the pressure applied to the chest of the patient by the responder during the chest compression. The pressure sensor may continuously measure the magnitude of the force applied to the chest of the patient over a given period, at a certain point in time, or at a plurality of time points over a given period. The pressure sensor may acquire the pressure sensor data and provide it to the processor. All or only some of the pressure sensor data may be provided to the processor. For example, the pressure sensor data may only be provided to the processor if the measured magnitude of the force changes by a predetermined amount. The pressure sensor may also be referred to as a force sensor.

The processor may be configured to determine whether the magnitude of the force is above a predetermined force threshold. The processor may be configured to determine whether a magnitude of the linear acceleration of the chest of the patient in the direction corresponding to the transverse plane of the patient is below a predetermined acceleration threshold which is less than the predetermined force threshold.

The linear acceleration of the chest of the patient in the direction corresponding to the transverse plane of the patient may be negative when the chest compression is applied and the magnitude of the linear acceleration may be lower than the predetermined acceleration threshold, which may be a negative number, when the chest compression is optimally applied, i.e. if the force of the chest compression is applied in a suitable direction which maximizes pumping of blood from the heart. The force measured by the pressure sensor may be positive when the chest compression is applied and the magnitude of the force may be above the predetermined force threshold when the chest compression is optimally applied. The predetermined force threshold may be positive and therefore may be larger than the predetermined acceleration threshold.

Thus, by monitoring the magnitude of the force and the magnitude of the linear acceleration in the direction corresponding to the transverse plane of the patient, it may be determined whether the chest compression is optimally applied. If the magnitude of the force exceeds the force threshold, yet the magnitude of the linear force does not fall below the (negative) acceleration threshold, then it may be determined that the chest compression is not optimally applied. This is because the magnitude of the applied force of the chest compression is high yet the magnitude of the linear acceleration is not equivalently high in the negative direction and so the force must be applied, at least partially, in a direction other than the direction corresponding to the transverse plane of the patient. Thus, the force profile may be determined by monitoring and analyzing the force measured by a pressure sensor and the linear acceleration of the chest of the patient in a direction corresponding to the transverse plane of the patient so as to determine whether the force of the chest compression is optimally applied.

The processor may be configured to determine a message indicating that the force of the chest compression is not optimally applied in response to the magnitude of the force being above the predetermined force threshold and the magnitude of the linear acceleration of the chest of the patient in the direction corresponding to the transverse plane of the patient being above the predetermined acceleration threshold. That is, if the magnitude of the force is above the predetermined force threshold and the magnitude of the linear acceleration is above the predetermined acceleration threshold, which may be a negative number, then the processor may determine that the chest compression is not optimally applied and may therefore determine a message to be communicated to the responder which reflects this. For example, the message may indicate that the force of the chest compression is applied in the wrong direction and the message may include a reminder of the direction of force that will likely result in optimum chest compression application, i.e. directly downward in a direction perpendicular to the surface that the patient is lying on

The chest compression may be one of a plurality of periodic chest compressions applied by the responder during CPR delivery. The sensor may be configured to acquire sensor data indicating the movement of the chest of the patient for each chest compression of the plurality of periodic chest compressions. The processor may be configured to determine a force profile of the force of each chest compression of the plurality of periodic chest compressions. The message communicated to the responder may provide feedback on the chest compression such that the responder may alter their arm positioning to improve subsequent chest compressions of the plurality of chest compressions.

The sensor may be positioned on the chest of the patient during the delivery of CPR such that the sensor may measure data indicative of the movement of the chest of the patient during the delivery of CPR, such as, for example, acceleration data. The sensor may be provided as a patch to be positioned on the chest of the patient. That is, the sensor may, at least partially be provided as a patch positioned on or attached to the chest of the patient. The patch may be provided with adhesive to attach the patch to the chest.

The sensor may also be provided as part of a CPR puck device used to assist with the delivery of CPR. The patch or CPR puck may be configured to be positioned between the chest of the patient and the hands of the responder during application of the chest compression by the responder, such that the hands of the responder engage with the patch or CPR puck and the force is applied to the patch or CPR puck. Thus, the patch or the CPR puck may be positioned on the chest of the patient at a location at which the responder engages with the chest when applying the chest compression.

The aspects of the CPR system may be provided as a single CPR device or may be provided as separate communicably coupled elements. Embodiments of the present invention therefore extend to a CPR device and a system comprising the CPR device and further relevant devices and/or elements. Features of the system aspects apply to the device aspects mutatis mutandis, and vice versa.

The present invention extends to method aspects corresponding to the device aspects.

According to an embodiment of another aspect, there is provided a method of assessing chest compressions applied to a patient by a responder during delivery of cardiopulmonary resuscitation, CPR, to the patient, the method comprising: acquiring sensor data indicating the movement of the chest of the patient due to a chest compression applied by the responder during CPR delivery; determining a force profile of the chest compression applied by the responder during CPR delivery using the acquired sensor data; determining a message to be communicated to the responder in accordance with the force profile; and outputting the message so as to communicate the message to the responder.

Thus, according to an embodiment of an aspect, a method of assessing the force applied to the chest of the patient may also be provided. The method utilizes sensor data indicative of the movement of the chest of the patient during a chest compression applied by a responder to determine a force profile of the chest compression. A message is determined based on the force profile and is output to be communicated to the responder. The force applied by the responder during the chest compression may be assessed and a message may be determined in accordance with the assessed force and delivered to the responder who applied the chest compression. By analyzing the chest compression and providing a corresponding message to the responder, subsequent chest compressions applied by the responder may be altered such that they result in more effective delivery of CPR.

Features and sub-features of the system aspects may be applied to the method aspects and vice versa.

The present invention extends to a computer program aspect which, when executed on a computing device, carries out a method, according to any of the method aspects of the invention or any combination thereof.

In particular, according to an embodiment of another aspect, there is provided a computer program which, when executed on a computing system, carries out a method of assessing chest compressions applied to a patient by a responder during delivery of cardiopulmonary resuscitation, CPR, to the patient, the method comprising: acquiring sensor data indicating the movement of the chest of the patient due to a chest compression applied by the responder during CPR delivery; determining a force profile of the chest compression applied by the responder during CPR delivery using the acquired sensor data; determining a message to be communicated to the responder in accordance with the force profile; and outputting the message so as to communicate the message to the responder.

Aspects of the invention, such as, for example, the processor, may be implemented in digital electronic circuitry, or in computer hardware, firmware, software, or in combinations of them. Aspects of the invention may be implemented as a computer program or computer program product, i.e., a computer program tangibly embodied in an information carrier, e.g., in a machine-readable storage device or in a propagated signal, for execution by, or to control the operation of, one or more hardware modules. A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a communication system environment. A computer program may be deployed to be executed on one module or on multiple modules at one site or distributed across multiple sites and interconnected by a communication network. Elements that are communicably coupled may be connected to the same network.

Aspects of the method steps of the invention may be performed by one or more programmable processors executing a computer program to perform functions of the invention by operating on input data and generating output. Aspects of the apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

It may therefore be seen that embodiments of the present invention may provide means for assessing chest compressions applied to a patient by a responder during delivery of CPR to the patient. Sensor data indicative of movement of the chest of the responder during a chest compression may be acquired and used to determine a force profile of the chest compression. The force profile may indicate a direction of the force applied during the chest compression. A message may then be determined using the force profile and the message may be output so as to be communicated to the responder. Accordingly, the chest compression applied by the responder may be analyzed and a message may be delivered to the responder based on the analysis. The analysis may, for example, determine that the direction of the applied force is not appropriate for achieving sufficient or optimum manual pumping of blood during CPR delivery and the message may therefore communicate this to the responder. By analyzing the chest compression and providing a corresponding message to the responder, subsequent chest compressions applied by the responder may be altered such that they result in more effective delivery of CPR. The CPR may therefore be more effectively applied and the benefit of CPR to the patient may be increased.

BRIEF DESCRIPTION OF THE DRAWINGS

Embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.

Fig. 1 is a block diagram of a cardiopulmonary resuscitation, CPR, system according to a general embodiment of the invention;
Fig. 2 is a flow chart of a method of assessing chest compressions applied during delivery of cardiopulmonary resuscitation, CPR, according to a general embodiment of the invention;
Fig. 3 is diagram indicating incorrect positioning of the arms of the responder during CPR delivery;
Fig. 4 is a diagram of a sensor according to an embodiment of an aspect of the invention;
Fig. 5(a) is a graph of example sensor data acquired during CPR delivery according to an embodiment of the invention; and
Fig. 5(b) is a graph of example sensor data acquired during CPR delivery according to an embodiment of the invention.

DETAILED DESCRIPTION OF EMBODIMENTS

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein may be used in the practice or testing of the embodiments.

Proper performance and delivery of CPR requires that the force applied by the responder's arm to the patient's chest during chest compressions be oriented directly downwards, with respect to the chest of the patient, so as to maximize cardiac blood ejection resulting from the chest compressions. If the force is not applied directly downwards with respect to the chest of the patient, then the cardiac massage may be less optimal and the success of maintaining adequate blood supply to the brain may be negatively impacted. In other words, a critical percent of the force applied by the responder for resuscitating the patient's blood circulation could be lost due to sub-optimal direction of the applied force relative to the heart's ejection pathway. Accordingly, improper application of force by the responder during the chest compressions of CPR can cause poor delivery of CPR and poor resulting blood flow, which may result in lower patient outcomes, such as, for example, neurological conditions. It is therefore important that the force applied to the patient during chest compressions of CPR delivery have appropriate orientation and direction so as to produce effective cardiac blood ejection.

Responders may be trained on how to properly orientate their arms during CPR delivery. However, in a real-world scenario, a responder may forget this training, may not be mindful of their arm positioning, may be under psychological stress and/or may lose the proper orientation and posture due to intense fatigue.

As discussed above, it is desirable to assess the application of chest compressions during CPR delivery to the patient by the responder so that the CPR may be more effectively applied and the benefit of CPR to the patient may be increased. Embodiments of the present invention provide means for assessing chest compressions applied to a patient by a responder during delivery of CPR to the patient. By analyzing the chest compression and providing a corresponding message to the responder, subsequent chest compressions applied by the responder may be altered such that they result in more effective delivery of CPR. Embodiments of the present invention may therefore provide means for constantly monitoring the responder's arm orientation and positioning, and for providing appropriate feedback on the arm orientation and positioning, which may, for example, instruct the responder on how to correct their arm orientation and positioning.

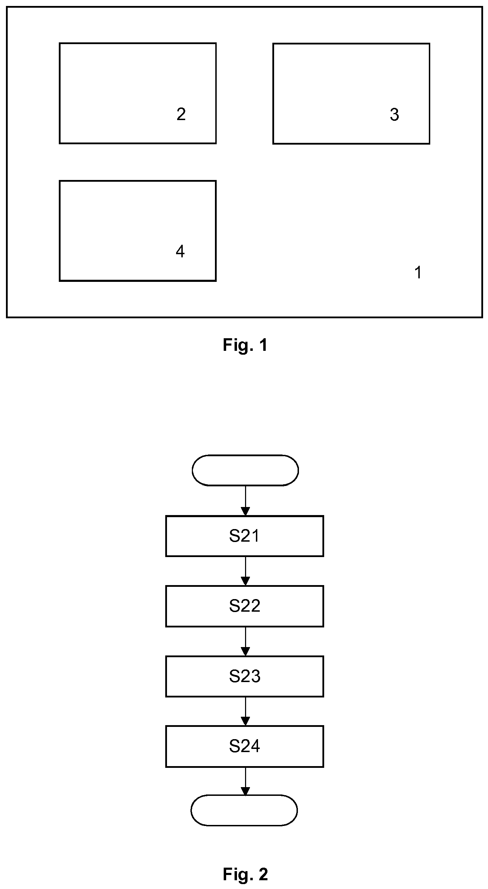

Fig. 1 shows a block diagram of a cardiopulmonary resuscitation, CPR, system according to a general embodiment of the invention. The CPR system 1 comprises a sensor 2, a processor 3 and an output 4. The sensor 2, the processor 3 and the output 4 may be communicably coupled with each other such that data may be transferred between the elements of the system. The sensor 2 acquires acquire sensor data indicating movement of the chest of the patient due to a chest compression applied by the responder during CPR delivery. The sensor 2 may therefore obtain sensor data for a measurement that indicates movement of the chest of the patient or may be used to determine data indicative of movement of the chest of the patient. The processor 3 determines a force profile of the chest compression applied by the responder during CPR delivery using the acquired sensor data and determines a message to be communicated to the responder in accordance with the force profile. The force profile may indicate a direction of the force applied during the chest compression and the message may include an instruction to alter the direction of the applied force in a subsequent chest compression during CPR delivery. The output 4 outputs the determined message so as to communicate the message to the responder.

Fig. 2 shows a flow chart of a method of assessing chest compressions applied to a patient by a responder during delivery of cardiopulmonary resuscitation, CPR, to the patient. At step S21, sensor data indicating the movement of the chest of the patient due to a chest compression applied by the responder during CPR delivery is acquired. The acquired sensor data is used at step S22 to determine a force profile of the chest compression applied by the responder during CPR delivery. A message to be communicated to the responder is determined in accordance with the force profile at step S23 and finally, at step S24, the message is output so as to be communicated to the responder.

Embodiments of the present invention may therefore assess the chest compression and provide feedback to the responder about the chest compression. Performance of improper CPR due to the responder's arm orientation relative to the patient may therefore be corrected by assessing the movement of the chest of the patient, determining the force profile and communicating a message to the responder in accordance with the force profile. For example, the force profile may indicate that the direction of the force of the chest compression is not downward on the patient's chest and a message may therefore be determined and output to instruct the responder to adjust their arm position so that the force is applied downward. The system may therefore provide up-to-date feedback on the applied force and the responder's arm orientation by continuously analyzing the acquired sensor data.

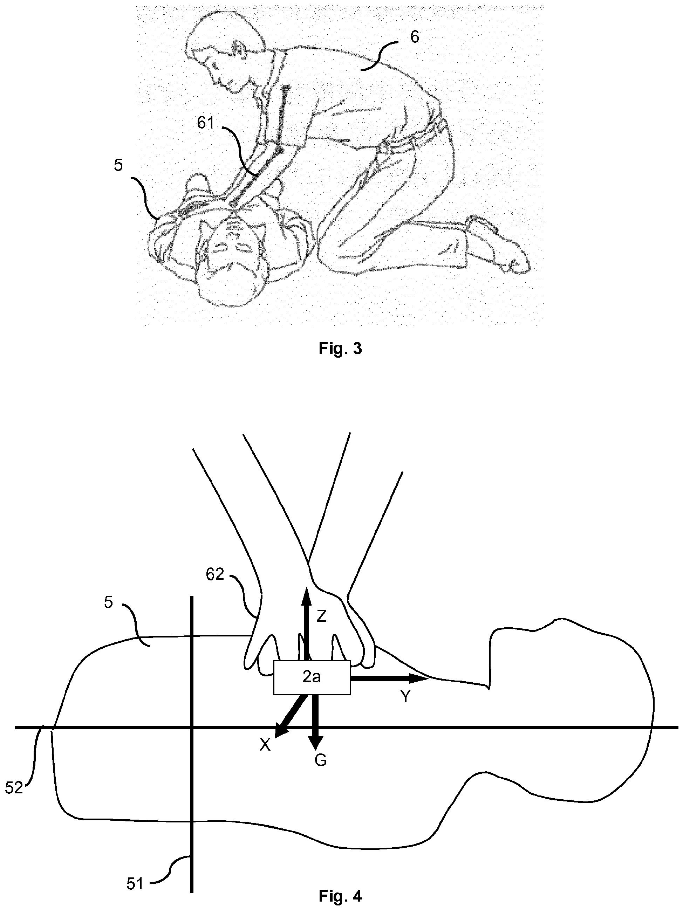

Fig. 3 is diagram indicating incorrect positioning of the arms of the responder during CPR delivery. As shown in Fig. 3, the responder 6 is applying a chest compression to the patient 5. The line 61 of the arm of the responder 6 is not vertically aligned such that the shoulder of the responder 6 is not positioned above the hands of the responder 6. Thus the positioning of the arms of the responder 6 relative to the patient 5 is improper and the force of the chest compression will not be applied directly downward. This will result in a reduction in the effectiveness of the CPR. Acceleration data acquired by the sensor of the CPR system will indicate movement of the chest of the patient 5 in one or more directions other than the direction aligned with the transverse plane of the patient 5 as a result of the improper line 61 of the arm of the responder 6. From this sensor data, the force profile may be determined which may indicate that the direction of the force is not downward and a corresponding message may be determined based on the force profile. For example, the message may instruct the responder 6 to lean forward such that the shoulders of the responder 6 are positioned above the hands and the line 61 of the arm of the responder 6 becomes vertical.

The sensor may be provided in a patch to be placed on the torso/chest of the patient. Determination of the force profile may be achieved by an algorithm which analyzes the measured sensor data to determine how the patient's chest moves in response to the force applied by the responder and thus to determine the direction of the force and the orientation/position of the responder's arm relative to the patient's chest. The output may be an audio-visual system that communicates a message to the responder and thereby may provide feedback to the responder on how to adjust his/her arm orientation relative to the chest of the patient to improve the effectiveness of the CPR.

The sensor may be an accelerometer and the sensor data may be acceleration data of the acceleration of the sensor measured at the chest of the patient. More specifically, the sensor may be a triaxial accelerometer configured to measure triaxial accelerometer data, i.e. acceleration in an x-axis direction, a y-axis direction and a z-axis direction.

An algorithm may be implemented by the processor to determine a force profile of the chest compression based on the triaxial accelerometer data. The algorithm may therefore estimate the direction of force being applied to the accelerometer and its relation relative to gravity, and thus feedback on the proper arm orientation may be provided to the responder in the message determined by the processor.

Fig. 4 is a diagram of a sensor according to an embodiment of an aspect of the invention. The sensor 2a shown in Fig. 4 is a triaxial accelerometer that measures acceleration in three orthogonal directions, corresponding to the x-axis X, the y-axis Y and the z-axis Z. As shown in Fig. 4, the z-axis Z is aligned with gravity G such that acceleration of the sensor in the negative z-axis direction Z corresponds to movement in the same direction as gravity G. Alignment of the z-axis Z and gravity G represents a situation in which the patient is lying on a flat surface. If the patient 5 is not lying on a flat surface such that the torso is inclined, the z-axis Z will not be directly aligned with gravity G. However, gravity G will always be most closely aligned with the z-axis Z, compared with the x-axis X and the y-axis Y. The z-axis Z is also aligned with the transverse plane 51 of the patient 5, which may also be referred to as an axial or horizontal plane and is perpendicular to the coronal or frontal plane 52 of the patient. The x-axis X and the y-axis Y correspond to the frontal plane 52 of the patient.

The triaxial accelerometer 2a of Fig. 4 may be used to provide CPR arm orientation feedback about the hands and arms of the responder 62. The accelerometer 2a measures acceleration of the device and the chest of the patient 5 in the three orthogonal directions. That is, measurements of the acceleration in the x-axis X (outside of the plane of the figure), the y-axis Y and the z-axis Z are acquired and a force profile of the chest compression may be derived from the measurements. Gravity G is used as reference to obtain proper orientation of the force being applied, such that the force profile may indicate the direction of the applied force with respect to gravity G. If the chest compression is properly applied, the direction of the force will be straight downward and aligned with gravity G (when the patient is lying on a flat surface). The majority of the measured acceleration should therefore only be in the negative z-axis Z direction. Thus, using accelerometer data the dominant axis on which the force is being applied may be estimated.

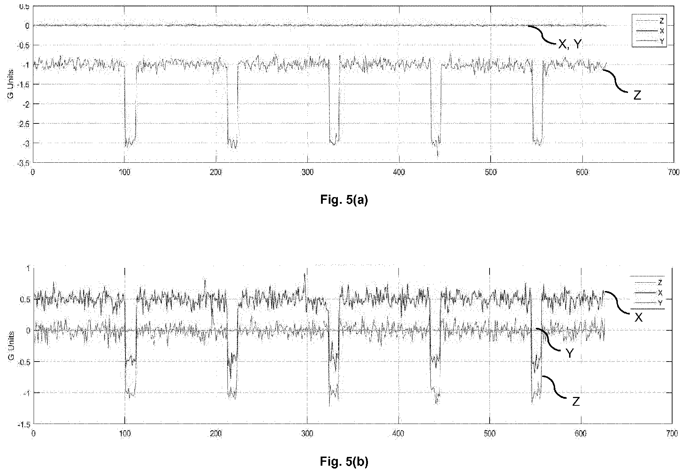

Fig. 5(a) shows a graph of example sensor data acquired during CPR delivery according to an embodiment of the invention. Such a graph may be considered as a force profile. The horizontal axis of the graph depicts time and the vertical axis of the graph depicts acceleration measured in units of G. The graph depicts the acceleration of the chest of the patient in the x-axis X, y-axis Y and z-axis Z directions. As shown in Fig. 5(a), the acceleration in the x-axis X and y-axis Y directions remains approximately equal to 0G throughout the CPR delivery and the acceleration in the z-axis Z direction periodically falls to approximately minus 3G. Thus, in Fig. 5(a) it can be determined that the force is correctly applied in the negative z-axis Z direction and that the positioning of the arm of the responder is proper. This graph therefore shows a simulation of correct CPR performance by the responder. The periodic drop in the z-axis Z direction represents periodic chest compressions applied by the responder. The raw accelerometer data may therefore be used to determine the axis on which the force is being applied and the acceleration should predominantly appear on the z-axis Z (aligned with gravity at -1).

Fig. 5(b) shows a graph of example sensor data acquired during CPR delivery according to an embodiment of the invention. Similarly to Fig. 5(a), the horizontal axis of the graph depicts time and the vertical axis of the graph depicts acceleration measured in units of G. In the graph of Fig. 5(b), acceleration is measured in both the x-axis X and z-axis Z directions and so it is evident that the force is not applied in the correct direction due to the dispersion of energy from the z-axis Z to the x-axis X and the arm positioning of the responder is not proper. This graph therefore shows a simulation of incorrect CPR performance, such as that shown in Fig. 3. The acceleration has been dispersed between the z-axis Z and the x-axis X. In general, deviations from the correct negative G on the z-axis Z indicates improper arm positioning and the message may be determined to provide feedback to the responder about proper arm positioning. The acceleration in the y-axis direction in Fig. 5(b) is approximately equal to 0G throughout the CPR delivery, indicating that the applied force is not dispersed in this direction.

The processor may use the trigonometric relationship between the angles to determine and provide proper angle feedback to the responder. The feedback may be provided as the message communicated by the output of the system. The output may, for example, be an LED display providing visual feedback to the responder.

For example, assuming the patient is lying down fully horizontal, and with Gz representing the component of acceleration due to gravity in the Z-axis we have:

$$\text{CPR Acceleration} = aCPR = \sqrt{ax^2 + ay^2 + (az-Gz)^2}$$

Where ax, ay, and az are the accelerations due to a CPR force in the x-axis, y-axis and z-axis directions, respectively. For the fully horizontal patient lying on a flat surface, the correct force should be applied in the z-axis direction, and can be estimated from:

$$aCPR\ correct = \ sqrt((az\text{-}Gz)^\wedge 2)\ /\ aCPR$$

Which represents percent of acceleration not aligned perpendicular to the plane of the patient's chest. Similarly, the equations above may be generalized to the case of a patient in an incline position (excluding a patient rotated on the x-axis or on his/her shoulder) i.e. a patient lying on their back but not on a flat surface, such that the z-axis is not aligned with gravity. With a short calibration phase for the estimation of gravity in the y and z components from the accelerometer data: Gy, Gz (with Gx=0 and G= sqrt(Gy^2 + Gz^2)). With these estimates, in place the equation above can be generalized to a patient plane that is inclined within the Z and Y plane:

$$Patient\ Z\text{-}angle = ThetaZ = arccos(Gz/G)$$

$$Patient\ Y\text{-}angle = ThetaY = arcsin(Gy/G)$$

The percent of force that is then being correctly applied to the patient (i.e. in the patient's Z plane) is then given by:

$$aCPR = sqrt(\ ax^\wedge 2 + (ay\text{-}Gy)^\wedge 2 + (az\text{-}Gz)^\wedge 2)$$

$$aCPR\ correct = \ sqrt((ay*sin(ThetaY)\text{-}Gy)^\wedge 2 + (az*cos(ThetaZ)\text{-}\ Gz)^\wedge 2)\ /\ aCPR$$

The processor may employ these equations in an algorithm to determine the force profile of the chest compression and thus the direction of the applied force, with respect to the torso of the patient and the inclination of the patient on a surface.

The sensor may also comprise a single axis accelerometer configured to measure the acceleration in the z-axis direction and a pressure sensor configured to measure the magnitude of the applied force. If the magnitude of acceleration in the negative z-axis direction does not correspond to the measurement of the magnitude of the force by the pressure sensor, then it may be determined that the force is incorrectly applied. That is, if the magnitude of the acceleration in the negative z-axis direction is low, then the measurement of the pressure sensor may be used to verify whether this is due to low overall force application, or application of the force in the wrong direction. Similarly, although not as accurate as a triaxial accelerometer, an accelerometer with an axis aligned with gravity and a second orthogonal axis may also be able to determine, at least in some cases, whether the force is applied in the correct direction. For example, an accelerometer measuring the acceleration in the y-axis and z-axis directions may be used.

As may be seen from the above, embodiments of the present invention may provide a system for assessing chest compressions applied to a patient by a responder during delivery of CPR to the patient. The assessment of the chest compressions may be used to determine a message to be output to the responder so that feedback regarding the chest compressions applied by the responder may be provided. The assessment may determine whether the force of the chest compression is improperly applied and may instruct the responder to alter the positioning of their arms with respect to the chest of the patient so as to properly apply the force. The CPR may therefore be more effectively applied and the benefit of CPR to the patient may be increased.

Although only a few exemplary embodiments have been described in detail above, those skilled in the art will readily appreciate that many modifications are possible in the exemplary embodiments without materially departing from the novel teachings and advantages of the embodiments of the present disclosure. The above-described embodiments of the present invention may advantageously be used independently of any other of the embodiments or in any feasible combination with one or more others of the embodiments.

Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements. In a device or apparatus claim enumerating several means, several of these means may be embodied by one and the same item of hardware. The mere fact that certain measures are recited in mutually different dependent claims does not indicate that a combination of these measures cannot be used to an advantage.

**Claims**

1. A cardiopulmonary resuscitation, CPR, system (1) for assessing chest compressions applied to a patient (5) by a responder (6) during delivery of CPR to the patient (5), the system (1) comprising:

   a sensor (2) configured to acquire sensor data indicating movement of the chest of the patient (5) due to a chest compression applied by the responder (6) during CPR delivery;
   a processor (3) configured to determine a force profile of the chest compression applied by the responder (6) during CPR delivery using the acquired sensor data; and
   an output (4) configured to output a message so as to communicate the message to the responder (6), wherein the processor (3) is configured to determine the message to be communicated to the responder (6) in accordance with the force profile.

2. The system (1) of claim 1, wherein the force profile indicates a direction of a force of the chest compression relative to gravity.

3. The system (1) of claim 1 or 2, wherein
   the sensor (2) is a triaxial accelerometer (2a) configured to measure linear acceleration of the chest of the patient (5) in three orthogonal directions; and
   one of the three orthogonal directions corresponds to a transverse plane (51) of the patient (5).

4. The system (1) of claim 3, wherein the processor (3) is configured to:

   analyze the sensor data of the linear acceleration of the chest of the patient (5) in the three orthogonal directions; and
   determine the direction of the force of the chest compression with respect to the three orthogonal directions.

5. The system (1) of claim 4, wherein the force profile indicates a direction of the force of the chest compression with respect to the three orthogonal directions.

6. The system (1) of any of claims 3 to 5, wherein the processor (3) is configured to:

   determine whether a magnitude of linear acceleration in a direction other than the direction corresponding to the transverse plane (51) of the patient (5) is below a first predetermined magnitude threshold; and
   determine whether a magnitude of linear acceleration in a direction other than the direction corresponding to the transverse plane (51) of the patient (5) is above a second predetermined magnitude threshold, which is greater than the first predetermined magnitude threshold.

7. The system (1) of claim 6, wherein the processor (3) is configured to determine a message indicating that the force of the chest compression is not optimally applied in response to the magnitude of linear acceleration in the direction other than the direction corresponding to the transverse plane (51) of the patient (5) being below the first predetermined magnitude threshold or the magnitude of linear acceleration in the direction other than the direction corresponding to the transverse plane (51) of the patient (5) being above the second predetermined magnitude threshold.

8. The system (1) of any of claims 3 to 7, wherein
   the sensor (2) is configured to:

   measure the linear acceleration of the chest of the patient (5) at a first time point; and
   measure the linear acceleration of the chest of the patient (5) at a second time point occurring later than the first time point; and

   the processor (3) is configured to:

determine a first relationship between the three orthogonal directions at the first time point;
determine a second relationship between the three orthogonal directions at the second time point;
calculate a difference between the first relationship and the second relationship; and
determine whether the difference exceeds a predetermined difference threshold; and
determine a message indicating that the force of the chest compression is not optimally applied in response to the difference exceeding the predetermined difference threshold.

9. The system (1) of any of claims 3 to 8, wherein
the sensor (2) further comprises a triaxial gyroscope configured to measure angular acceleration of the chest of the patient (5) about the transverse plane (51) of the patient (5); and
the processor (3) is configured to:

analyze the sensor data of the angular acceleration of the chest of the patient (5); and
determine the direction of the force of the chest compression with respect to the angular acceleration and linear acceleration of the chest of the patient (5).

10. The system (1) of claim 1, wherein
the sensor (2) comprises:

an accelerometer configured to measure linear acceleration of the chest of the patient (5) in a direction corresponding to a transverse plane (51) of the patient (5); and
a pressure sensor configured to measure a magnitude of the force of the chest compression; and

the force profile indicates linear acceleration of the chest of the patient (5) in the direction corresponding to the transverse plane (51) of the patient (5) in accordance with the magnitude of the force.

11. The system (1) of claim 10, wherein the processor (3) is configured to:

determine whether the magnitude of the force is above a predetermined force threshold; and
determine whether a magnitude of the linear acceleration of the chest of the patient (5) in the direction corresponding to the transverse plane (51) of the patient (5) is below a predetermined acceleration threshold which is less than the predetermined force threshold.

12. The system (1) of claim 11, wherein the processor (3) is configured to configured to determine a message indicating that the force of the chest compression is not optimally applied in response to the magnitude of the force being above the predetermined force threshold and the magnitude of the linear acceleration of the chest of the patient (5) in the direction corresponding to the transverse plane (51) of the patient (5) being above the predetermined acceleration threshold.

13. The system (1) of any preceding claim, wherein
the chest compression is one of a plurality of periodic chest compressions applied by the responder (6) during CPR delivery;
the sensor (2) is configured to acquire sensor data indicating the movement of the chest of the patient (5) for each chest compression of the plurality of periodic chest compressions; and
the processor (3) is configured to determine a force profile of the force of each chest compression of the plurality of periodic chest compressions.

14. A method of assessing chest compressions applied to a patient (5) by a responder (6) during delivery of cardiopulmonary resuscitation, CPR, to the patient (5), the method comprising:

acquiring (S21) sensor data indicating the movement of the chest of the patient (5) due to a chest compression applied by the responder (6) during CPR delivery;
determining (S22) a force profile of the chest compression applied by the responder (6) during CPR delivery using the acquired sensor data;
determining (S23) a message to be communicated to the responder (6) in accordance with the force profile; and
outputting (S24) the message so as to communicate the message to the responder (6).

15. A computer program which, when executed on a computing system, carries out a method of assessing chest compressions applied to a patient (5) by a responder (6) during delivery of cardiopulmonary resuscitation, CPR, to the patient (5), the method comprising:

acquiring (S21) sensor data indicating the movement of the chest of the patient (5) due to a chest compression applied by the responder (6) during CPR delivery;
determining (S22) a force profile of the chest compression applied by the responder (6) during CPR delivery using the acquired sensor data;
determining (S23) a message to be communicated to the responder (6) in accordance with the force profile; and
outputting (S24) the message so as to communicate the message to the responder (6).

**Fig. 1**

**Fig. 2**

**Fig. 3**

**Fig. 4**

**Fig. 5(a)**

**Fig. 5(b)**

Europäisches
Patentamt

European
Patent Office

Office européen
des brevets

## EUROPEAN SEARCH REPORT

Application Number

EP 19 17 9596

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/165634 A1 (UNIV ARIZONA [US]) 13 September 2018 (2018-09-13) * claims 1-4 * * figures 1A-1G, 3 * * page 2, line 12 - line 15 * * page 4, line 23 - line 28 * * page 5, line 15 - line 18 * * page 6, line 24 - line 25 * * page 8, line 19 - line 20 * * page 11, line 13 - line 18 * * page 12, line 19 - line 20 * * page 13, line 7 - line 8 * * page 17, line 23 - line 30 * * page 20, line 7 - line 12 * * page 5, line 5 - line 9 * * page 21, line 10 - line 13 * ----- | 1-15 | INV. G16H20/40 |

TECHNICAL FIELDS
SEARCHED       (IPC)

G16H

The present search report has been drawn up for all claims

| Place of search | Date of completion of the search | Examiner |
|---|---|---|
| Munich | 29 November 2019 | Beligny, Samuel |

## ANNEX TO THE EUROPEAN SEARCH REPORT
## ON EUROPEAN PATENT APPLICATION NO.

EP 19 17 9596

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2019

| Patent document cited in search report | Publication date | Patent family member(s) | Publication date |
|---|---|---|---|
| WO 2018165634 A1 | 13-09-2018 | NONE | |

For more details about this annex : see Official Journal of the European Patent Office, No. 12/82